Europäisches Patentamt

European Patent Office

Office européen des brevets

(19)

(11) Veröffentlichungsnummer: **0 033 164**
A2

(12) # EUROPÄISCHE PATENTANMELDUNG

(21) Anmeldenummer: 81100650.1

(22) Anmeldetag: 29.01.81

(51) Int. Cl.³: **A 61 K 7/06**
A 61 K 31/565, A 61 K 31/57

(30) Priorität: 29.01.80 DE 3003036

(43) Veröffentlichungstag der Anmeldung:
05.08.81 Patentblatt 81/31

(84) Benannte Vertragsstaaten:
AT BE CH DE FR GB IT LI LU NL SE

(71) Anmelder: von Kistowski, Irmgard, Dr.
Kurfürstenstrasse 50
D-5000 Köln 1(DE)

(72) Erfinder: von Kistowski, Irmgard, Dr.
Kurfürstenstrasse 50
D-5000 Köln 1(DE)

(74) Vertreter: Werner, Hans Karsten, Dr. et al,
Deichmannhaus am Hauptbahnhof
D-5000 Köln 1(DE)

(54) Mittel zur Verhinderung und Beseitigung von Glatzen.

(57) Mittel zur Verhinderung und Beseitigung von Glatzen, welches natürliche und/oder voll- oder halbsynthetische Östrogene in einer Dosierung von 1/4 bis 1/20 der üblichen Dosierung der natürlichen und/oder voll- oder halbsynthetischen Östrogene enthält, wobei gewünschtenfalls auch zusätzlich Gestagene enthalten sein können.

EP 0 033 164 A2

Mittel zur Verhinderung und Beseitigung von Glatzen
------------------------------------------------------------

Gegenstand der vorliegenden Erfindung ist ein neues Mittel zur Verhinderung des Haarausfalls und der Anregung des Haarwuchses auf dem Kopf erwachsener Männer, d.h. der Verhinderung und Beseitigung von Glatzen. Es werden seit Jahrzehnten die verschiedensten vorzugsweise äußerlich anzuwendenden Mittel zur Verhinderung des Haarausfalls oder gar zur Anregung des Haarwuchses auf bereits bestehenden Glatzen angeboten und vertrieben. Der Erfolg dieser Mittel ist bekanntlich außerordentlich gering, so daß diese Mittel wohl auch von zweifelhaftem Wert sind. Neben Pflanzenextrakten, Schwefelpräparaten und Cortison-Präparaten hat man auch schon versucht, diesen extern anzuwendenden Östrogene zuzufügen. Aber auch dieses Präparat führt in der Praxis nicht zu dem gewünschten Erfolg.

Die vorliegende Erfindung hat sich die Aufgabe gestellt, ein Mittel zu entwickeln, welches tatsächlich wirksam ist und dabei praktisch keine unerwünschten Nebenwirkungen zeigt oder erwarten läßt. Die Aufgabe wird durch ein neues Mittel gelöst, welches natürliche und/oder voll- oder halbsynthetische Östrogene, ggf. auch Gestagene in sehr geringer Dosierung enthält. Diese Wirkstoffe werden normalerweise nur Frauen verabfolgt sowie in sehr hoher Dosierung auch Männern bei inoperablen Prostatakarzinomen. In der üblichen oder gar sehr hohen Dosierung treten erhebliche und schwerwiegende Nebenwirkungen auf, so daß ein erhebliches Vorurteil dagegen bestand, diese Wirkstoffe überhaupt bei Männern einzusetzen. Insbesondere bestand ein schwerwiegendes Vorurteil dagegen, sie gesunden Männern zu verordnen, um hiermit in erster Linie einen kosmetischen Effekt zu erreichen. Keinesfalls vorhersehbar war, daß die Verhinderung und Beseitigung von Glatzen bereits durch so minimale Mengen der Östrogene erreicht werden kann. Bei diesen minimalen Dosierungen werden nämlich die üblichen Nebenwirkungen wie Potenzstörungen, Gewichtszunahme, Venenerkrankung und Übelkeit nicht mehr beobachtet. Gleichwohl hat sich gezeigt, daß in allen Fällen der Behandlung bereits nach drei bis vier Wochen der Haarausfall und die Schuppenbildung zum Stillstand kommt und nach ca. acht bis zwölf Wochen der Haarwuchs neu einsetzt.

Nebenwirkungen wurden in keinem Fall der Behandlung beobachtet. Dieser Befund ist besonders überraschend, da Östrogene auch in relativ hoher Dosierung bei äußerlicher Anwendung offensichtlich keinen Einfluß auf den Haarwuchs des erwachsenen Mannes haben. Es ist deshalb auch noch nicht geklärt, auf welchem Wirkmechanismus dieser neue Effekt der Östrogene zurückzuführen ist. Die bisherigen Befunde sprechen jedoch dafür, daß es sich um einen genbedingten leichten Östrogenmangel handelt, der zum Haarausfall und zur Glatzenbildung führt. Es ist nämlich bekannt, daß die Neigung zur Glatzenbildung und die Glatzenbildung als solche von Erbfaktoren beeinflußt wird. Nicht bekannt war hingegen, daß es sich hierbei u.U. nur um einen geringfügigen Östrogenmangel handelt, der erfindungsgemäß leicht und ohne Nebenwirkungen behandelt werden kann, indem man den natürlichen Östrogenspiegel bei Männern geringfügig erhöht.

Zur Behandlung kommen prinzipiell alle natürlichen, vollsynthetischen und halbsynthetischen Östrogene und Gestagene infrage, wobei sie je nach ihrer Aktivität dosiert werden müssen. Da die natürlichen Östrogene nur relativ schwach wirksam sind und deshalb in Tagesdosen von etwa 1 bis 1,5 mg appliziert werden, reichen für die Glatzenverhinderung und -beseitigung bereits Mengen von 0,05 bis 0,25 mg pro Tag aus. Die halbsynthetischen Östrogene sind wirksamer und werden daher bei Frauen normalerweise in einer Menge von 0,05 mg appliziert. Erfindungsgemäß reichen somit 0,002 bis 0,01 mg pro Tag, vorzugsweise werden sie in einer Menge von 0,005 mg pro Tag eingesetzt. In einigen leichteren Fällen wird man auch noch geringere Dosen applizieren können. Bei wesentlich höheren Dosen hingegen ist wohl mit den bekannten Nebenwirkungen zu rechnen.

Es ist bisher noch nicht erklärbar, worauf es zurückzuführen ist, daß der Zusatz einer geringen Menge Gestagne zu den Östrogenen den Effekt der Östrogene offensichtlich verstärkt, ohne jedoch die Nebenwirkungen der Östrogene in Erscheinung treten zu lassen. Vorzugsweise werden daher die Östrogene zusammen mit einer entsprechend geringen Menge Gestagen zur Anwendung gebracht.

Als besonders geeignetes halbsynthetisches Östrogen hat sich Ethinylestradiol bewährt, dem ggf. Lynestrenol zugefügt ist. Als Tagesdosis empfehlen sich je nach Körpergewicht des Patienten und der Schwere des Falles Mengen von 0,003 bis 0,006 mg pro Tag Ethinylestradiol und 0,15 bis 0,3 mg Lynestrenol.

Diese Wirkstoffe sind bisher noch nie in so niedriger Dosierung zu Tabletten oder Dragees verarbeitet worden. Die erfindungsgemäßen Mittel enthalten daher die bekannten Wirkstoffe zusammen mit den üblichen Hilfs- und Füllstoffen und können in Form von Tabletten oder Dragees hergestellt werden. Als Füll- und Hilfsstoffe kommen alle bisher für die Verarbeitung von natürlichen und/oder voll-oder halbsynthetischen Östrogenen und ggf. Gestagenen verwendeten und n verwendbaren Hilfsstoffe infrage. Bei der Verarbeitung zu Dragees gestaltet sich die Einnahme angenehmer, so daß die Drageeform zu bevorzugen ist. Es ist zwar prinzipiell möglich, die Tagesdosis in zwei oder drei Portionen zu verabfolgen und dementsprechend niedriger dosierte Tabletten und Dragees herzustellen. Da jedoch die Blut- und Gewebespiegel nur geringen Schwankungen im Tagesverlauf unterliegen, genügt es völlig, die Tagesdosis in Form einer Tablette oder eines Dragees zu verabfolgen.

In dem nachfolgenden Beispiel ist die erfindungsgemäße Verwendung der Östrogene bei beginnender und bereits vorhandener Glatze beschrieben.

Beispiel
--------

Handelsübliche Tabletten enthaltend pro Tablette 0,05 mg Ethinylestradiol und 2,5 mg Lynestrenol wurden in 8 etwa gleich große Teile zerbrochen. Täglich mit dem Frühstück wurde ein Achtel Tablette eingenommen. Die Dosierung betrug somit unter Berücksichtigung der unvermeidlichen Verluste durch Staub und Krümel etwa 0,005 mg Ethinylestradiol und 0,25 mg Lynestrenol pro Tag. Bereits nach 3 bis 4 Wochen war die Schuppenbildung, der deutliche Haarausfall und die Glatzenbildung gestoppt. Nach 8 bis 12 Wochen setzte auf den bereits kahlen Stellen deutlicher neuer Haarwuchs ein. Einige Monate später wiesen alle bisher entsprechend behandelten Männer wieder vollen Haarwuchs auf, sofern noch die Haarwurzeln vorhanden waren. Ähnliche Ergebnisse wurden mit natürlichen und halbsynthetischen Östrogenen ohne Zusatz von Gestagenen erzielt. Bei der niedrigen Dosierung der Wirkstoffe wurde in keinem Fall eine der üblichen Nebenwirkungen von Östrogenbehandlungen bei Männern beobachtet.

Für die praktische Anwendung des neuen Mittels ist es jedoch dringend zu empfehlen, Tabletten oder Dragees mit entsprechend niedriger Dosierung herzustellen, so daß diese unzerteilt täglich mit eingenommen werden können.

1. Mittel zur Verhinderung und Beseitigung von Glatzen enthaltend natürliche und/oder voll- oder halbsynthetischen Östrogene in einer Dosierung von 1/4 bis 1/20, vorzugsweise 1/8 bis 1/10 der üblichen Dosierung der natürlichen und/oder voll- oder halbsynthetischen Östrogene.

2. Mittel gemäß Anspruch 1, dadurch gekennzeichnet, daß es zusätzlich Gestagene enthält.

3. Mittel gemäß Anspruch 1 oder 2, dadurch gekennzeichnet, daß es voll- oder halbsynthetische Östrogene in einer Menge von 0,002 bis 0,01 mg enthält.

4. Mittel gemäß Anspruch 1 oder 2, dadurch gekennzeichnet, daß es natürliche Östrogene in einer Menge von 0,05 bis 0,25 mg enthält.

5. Mittel gemäß Anspruch 2 und 3, dadurch gekennzeichnet, daß es Gestagene in einer Menge von 0,05 bis 0,5 mg enthält.

6. Mittel gemäß Ansprüchen 1 bis 3, dadurch gekennzeichnet, daß es 0,003 bis 0,006 mg Ethinylestradiol und 0,15 bis 0,3 mg Lynestrenol enthält.

7. Verwendung von natürlichen und/oder voll- oder halbsynthetischen Östrogenen und ggf. Gestagenen zur Verhinderung und Beseitigung von Glatzen.

8. Verwendung von Ethinylestradiol in einer Dosierung von 0,003 bis 0,006 mg und Lynestrenol in einer Dosierung von 0,15 bis 0,3 mg zur Verhinderung und Beseitigung von Glatzen.